# EUROPÄISCHE PATENTANMELDUNG

(11) **EP 1 081 229 A1**
(43) Veröffentlichungstag der Anmeldung: **07.03.2001**
(21) Anmeldenummer: 00117247.7
(22) Anmeldetag: 14.08.2000
(51) Int. Cl.: C12P 7/52, C12P 7/40, C12P 11/00, C12N 1/20

(54) **Fermentatives Verfahren zur Gewinnung natürlicher aromatischer, aliphatischer und Thiocarbonsäuren und Mikroorganismus dafür**

(30) Priorität: 25.08.1999 EP 99116711
(71) Anmelder: HAARMANN & REIMER GMBH, 37601 Holzminden (DE)
(72) Erfinder: Rabenhorst, Jürgen, Dr., 37671 Höxter (DE); Gatfield, Ian-Lucas, Dr., 37671 Höxter (DE); Hilmer, Jens-Michael, Dr., 37671 Höxter (DE)
(74) Vertreter: Mann, Volker, Dr.

(57) **Zusammenfassung**

Die vorliegende Erfindung betrifft ein Verfahren zur Herstellung von aliphatischen, aromatischen und Thiocarbonsäuren, wobei Bakterien der Gattung Gluconobacter enthaltende Kulturen eingesetzt werden.

## Beschreibung

Die vorliegende Erfindung betrifft ein biologisches Verfahren zur Herstellung verschiedener Carbonsäuren durch die enzymatische Oxidation der entsprechenden Alkohole mit Alkoholoxidase in sehr hohen Ausbeuten. Die Alkoholoxidase wird von Bakterien der Gattung Gluconobacter, bevorzugt der Spezies Gluconobacter sp. HR 101 (DSM 12884), gebildet. So werden beispielsweise Benzoesäure aus Benzylalkohol, Buttersäure aus n-Butanol, Isobuttersäure aus Isobutanol, Isovaleriansäure aus Isoamylalkohol, 2-Methylbuttersäure aus 2-Methylbutanol, 3-Methylthiopropionsäure aus 3-Methylthiopropanol, Phenylessigsäure aus Phenylethanol, Propionsäure aus Propanol und Zimtsäure aus Zimtalkohol hergestellt.

Neben dem lange bekannten Verfahren der Essigherstellung durch Oxidation von Ethanol zu Essigsäure mit Bakterien der Gattung Acetobacter, gibt es auch einige Verfahren zur Herstellung von einigen Carbonsäuren mit Bakterien der Gattungen Acetobacter oder Gluconobacter oder mit Hefen.

So beschreibt die DE 3713668 die Herstellung aliphatischer Carbonsäuren durch mikrobielle Oxidation aliphatischer Alkohole mit Bakterien der Spezies Gluconobacter roseus. Die Alkohole wurden dabei nach einer Wachstumsphase von über 24 Stunden direkt in das Kulturmedium mit dem Organismus gegeben. Der bevorzugte pH-Bereich wurde mit 4 bis 4,5 angegeben. Es wurden nur geringe Ausbeuten von 13 g n-Buttersäure/l, 21 g Isobuttersäure/l, 7 g 2-Methylbuttersäure/l und 17 g 3-Methylbuttersäure/l Fermentationslösung erhalten.

In der DE 195 03 598 wird ein Verfahren zur Herstellung von Propionsäure oder Buttersäure bzw. deren Salze beschrieben. Sie verwenden ein Bakterium der Spezies Gluconobacter oxydans. Nach 9 bis 10 stündiger Kultivation wurde n-Propanol oder n-Butanol portionsweise in Abhängigkeit vom pO₂-Wert wiederholt zugegeben. Sie erzielten damit Ausbeuten von 43,7 g/L Propionsäure und 49 g/l Buttersäure.

In der EP 0563346 wird ein Verfahren zur Herstellung von Carbonsäuren durch Oxidation entsprechender Alkohole oder Aldehyde mit Hilfe einer Hefe der Gattung Saccharomyces, Hansenula, Pichia, Candida oder Kluyvermyces beschrieben. Nachteilig ist hier, dass mit den Hefen nur niedrige Produktkonzentrationen erhalten werden, sehr hohe Biomassekonzentrationen eingesetzt werden müssen und lange Prozesszeiten. So wurden nach vier Tagen nur weniger als 0,6 g/L 3-Methylthiopropansäure erhalten, und für die 90 %ige Umsetzung von 0,01 % Isoamylalkohol wurden 6 Tage benötigt.

In J. Chem.Tech.Biotechnol. 1997, 68, 214-218 wird die Biotransformation einiger aliphatischer Alkohole und 2-Phenylethanol in die entsprechenden Säuren mit Bakterien der Spezies Acetobacter aceti beschrieben. Nachteilig sind auch hier die geringen erhaltenen Produktkonzentrationen. So wurde die höchste beschriebene Produktkonzentration bei der Oxidation von Butanol zu Buttersäure mit 39,3 g/l nach 60 Stunden angegeben.

In J. Chem.Tech.Biotechnol. 1997, 70, 294-298 wird das Bacterium Acetobacter pasteurianus bei der oxidativen Herstellung bestimmter Carbonsäuren beschrieben. Von Nachteil ist hier die Verwendung von Air-lift Bioreaktoren, da durch den hohen Luftstrom, der für die Belüftung und Durchmischung der Kultur notwendig ist größere Mengen der flüchtigen Edukte und Produkte ausgetragen werden. Die aus diesem Grund dahinter angeschlossene Kühlfalle mit Flüssigstickstoff ist großtechnsich nicht praktikabel.

Es wurde eine Verfahren zur Herstellung von aliphatischen, aromatischen und Thiocarbonsäuren in Bioreaktoren gefunden, das dadurch gekennzeichnet ist, dass Bakterien der Gattung Gluconobacter enthaltende Kulturen eingesetzt werden.

Überraschend lassen sich mit den neuen Organismen der Gattung Gluconobacter sehr hohe Ausbeuten nicht nur an aliphatischen-, sondern auch an aromatischen- und thio-Carbonsäuren erzielen. Dies gilt sowohl in Hinsicht auf die Produktkonzentration in der Lösung, die prozentuale molare Umsetzung der Edukte als auch in Bezug auf die Raum-Zeit-Ausbeute. Dabei ist für den Prozess neben der Medienzusammensetzung und dem auf pH 6,4 gehaltenen pH-Wert, insbesondere die Art der kontinuierlichen Zugabe des Substrats von Bedeutung.

Als Bakterien werden bevorzugt für das erfindungsgemäße Verfahren Bakterien des Typs Gluconobacter sp. HR 101 (DSM 12884) eingesetzt.

Bevorzugt wird das Bakterium in Reinkultur eingesetzt.

Als Nährmedium für die erfindungsgemäß eingesetzten Organismen kommen synthetische, halbsynthetische oder komplexe Kulturmedien in Betracht. Diese können kohlenstoffhaltige und stickstoffhaltige Verbindungen, anorganische Salze, gegebenenfalls Spurenelemente sowie Vitamine enthalten.

Als kohlenstoffhaltige Verbindungen können Kohlenhydrate, Kohlenwasserstoffe oder organische Grundchemikalien in Betracht kommen. Beispiele für vorzugsweise verwendbare Verbindungen sind Zucker, Alkohole bzw. Zuckeralkohole, organische Säuren oder komplexe Gemische.

Als Zucker kommt vorzugsweise Glucose in Betracht. Als organische Säuren können vorzugsweise Zitronensäure oder Essigsäure zum Einsatz kommen. Zu den komplexen Gemischen zählen z. B. Malzextrakt, Hefeextrakt, Casein oder Caseinhydrolysat.

Als stickstoffhaltige Substrate kommen anorganische Verbindungen in Betracht. Beispiele hierfür sind Nitrate und Ammoniumsalze. Ebenso können organische Stickstoffquellen zum Einsatz kommen. Hierzu zählen Hefeextrakt, Sojamehl, Casein, Caseinhydrolysat und Maisquellwasser.

Zu den einsetzbaren anorganischen Salzen zählen beispielsweise Sulfate, Nitrate, Chloride, Carbonate und Phosphate. Als Metalle enthalten die genannte Salze vorzugsweise Natrium, Kalium, Magnesium, Mangan, Calcium, Zink und Eisen.

Die Temperatur für die Kultivierung liegt vorzugsweise im Bereich von 10 bis 40°C. Besonders bevorzugt ist der Bereich von 20 bis 35°C.

Der pH-Wert des Mediums beträgt bevorzugt 4 bis 8. Besonders bevorzugt ist der Bereich von 6,2 bis 6,5.

Grundsätzlich können für die Durchführung des erfindungsgemäßen Verfahrens alle dem Fachmann bekannten Bioreaktoren eingesetzt werden. Vorzugsweise kommen alle für Submersverfahren geeigneten Vorrichtungen in Betracht. Das heißt, es können erfindungsgemäß Gefäße ohne oder mit mechanischer Mischeinrichtung eingesetzt werden. Zu ersteren zählen z. B. Schüttelapparaturen, Blasensäulen- oder Schlaufenreaktoren. Zu letzteren gehören vorzugsweise alle bekannten Vorrichtungen mit Rührern in beliebiger Gestaltung.

Das erfindungsgemäße Verfahren kann kontinuierlich oder diskontinuierlich durchgeführt werden. Die Dauer der Fermentation bis zum Erreichen einer maximalen Produktmenge hängt von der speziellen Art des eingesetzten Organismus ab. Grundsätzlich liegen die Zeiten der Fermentation jedoch zwischen 2 und 200 Stunden.

Aliphatische Carbonsäuren für das erfindungsgemäße Verfahren sind Buttersäure, Isobuttersäure, Isovaleriansäure, 2-Methylbuttersäure, und Propionsäure.

Aromatische Carbonsäuren für das erfindungsgemäße Verfahren sind Benzoesäure, Phenylessigsäure und Zimtsäure.

Eine Thiocarbonsäure für das erfindungsgemäße Verfahren ist 3-Methylthiopropionsäure.

Bevorzugt werden nach dem erfindungsgemäßen Verfahren Buttersäure, Isobuttersäure, Isovaleriansäure, 2-Methylbuttersäure, Propionsäure, Phenylessigsäure und 3-Methylthiopropionsäure umgesetzt.

Insbesondere bevorzugt werden nach dem erfindungsgemäßen Verfahren Isobuttersäure, Isovaleriansäure, 2-Methylbuttersäure und Phenylessigsäure umgesetzt.

Im folgenden wird die Erfindung unter Bezugnahme auf Beispiele näher erläutert:

### Beispiele

### Beispiel 1 - Herstellung der Vorkultur

Ein 500 ml Erlenmeyerkolben mit seitlichem Einstich wird mit 100 ml sterilem Medium, bestehend aus 1,25 g D-Mannit und 0,75 g Hefeextrakt bei pH 6,5, mit 0,9 ml einer Glycerinkultur von Gluconobacter sp. HR 101 (DSM 12884) angeimpft. Der Kolben wird für 16 Stunden auf der rotierenden Schüttelmaschine bei 30 °C und 140 Upm inkubiert. Die Keimzahl der Vorkultur beträgt ca. 2 x 10⁹ KBE/ml.

### Beispiel 2 - Herstellung von natürlicher n-Buttersäure aus natürlichem n-Butanol

Es werden 125 g Mannit und 75 g Hefeextrakt in 9,9 l Wasser in einem 10 l Fermenter gelöst, 10 ml Antischaummittel hinzu gegeben und der pH-Wert auf 6,3 eingestellt. Das so hergestellte Medium wird für 30 Minuten bei 121°C sterilisiert.

Die Drehzahl des Rührers beträgt 500 Upm, die Belüftung 5 Nl/min; die Temperatur 30°C. Nach Einstellung dieser Parameter wird die Vorkultur gemäß Beispiel 1 zum Animpfen verwendet.

Nach 17 Stunden Fermentationszeit wird mit der Zugabe von n-Butanol über eine Pumpe begonnen. Die Dosierung des Substrats wird über einen Flow-Controller gesteuert. n-Butanol wird gemäß folgendem Flow-Profil zugegeben:

| Fermentationszeit | Flussrate |
|---|---|
| 0 h | 0 g/lh |
| 17 h | 1,0 g/lh |
| 20 h | 4,0 g/lh |
| 27 h | 3,0 g/lh |
| 30 h | 2,5 g/lh |
| 35 h | 2,0 g/lh |
| 50 h | 1,5 g/lh |
| 50,5 h | 2,0 g/lh |
| 53 h | 1,5 g/lh |
| 57 h | 1,0 g/lh |
| 63 h | 0 g/lh |
| 66 h | 1,0 g/lh |
| 68 h | 1,5 g/lh |
| 71 h | 1,0 g/lh |
| 73 h | 0 g/lh |

Während des Feedings wird der pH-Wert im Bereich von 6,2 ― 6,4 mit NH₄⁺ konstant gehalten.

Die Fermentation ist nach 74 Stunden beendet. Die Endkonzentration an n-Buttersäure beträgt laut HPLC-Analytik 95 g/l. Die molare Umsetzung beträgt knapp 90 %.

### Beispiel 3 - Herstellung von natürlicher Isobuttersäure aus Isobutanol

Es werden 125 g Mannit und 75 g Hefeextrakt in 9,9 l Wasser in einem 10 l Fermenter gelöst, 10 ml Antischaummittel hinzu gegeben und der pH-Wert auf 6,3 eingestellt. Das so hergestellte Medium wird für 30 Minuten bei 121°C sterilisiert.

Die Drehzahl des Rührers beträgt 500 Upm, die Belüftung 5 Nl/min; die Temperatur 30°C. Nach Einstellung dieser Parameter wird die Vorkultur gemäß Beispiel 1 zum Animpfen verwendet.

Nach 22,5 Stunden Fermentationszeit wird mit der Zugabe von Isobutanol über eine Pumpe begonnen. Die Dosierung des Substrats wird über einen Flow-Controller gesteuert. Isobutanol wird gemäß folgendem Flow-Profil zugegeben:

| Fermentationszeit | Flussrate |
|---|---|
| 0 h | 0 g/lh |
| 22,5 h | 1,0 g/lh |
| 23,5 h | 4,0 g/lh |
| 30 h | 3,0 g/lh |
| 35 h | 2,5 g/lh |
| 50 h | 2,0 g/lh |
| 50,3 h | 2,5 g/lh |
| 53 h | 1,5 g/lh |
| 58 h | 1,0 g/lh |
| 60 h | 0 g/lh |
| 64 h | 1,0 g/lh |
| 67 h | 0 g/lh |
| 68 h | 1,5 g/lh |
| 73 h | 1,0 g/lh |
| 74 h | 0 g/lh |

Während des Feedings wird der pH-Wert im Bereich von 6,2 ― 6,4 mit NH₄⁺ konstant gehalten.

Die Fermentation ist nach 74 Stunden beendet. Die Endkonzentration an Isobuttersäure beträgt laut HPLC-Analytik 92,7 g/l. Die molare Umsetzung beträgt knapp 88 %.

### Beispiel 4 - Herstellung von natürlicher 2-Methylbuttersäure aus 2-Methylbutanol

Es werden 125 g Mannit und 75 g Hefeextrakt in 9,9 l Wasser in einem 10 l Fermenter gelöst, 10 ml Antischaummittel hinzu gegeben und der pH-Wert auf 6,3 eingestellt. Das so hergestellte Medium wird für 30 Minuten bei 121°C sterilisiert.

Die Drehzahl des Rührers beträgt 500 Upm, die Belüftung 5 Nl/min; die Temperatur 30°C. Nach Einstellung dieser Parameter wird die Vorkultur gemäß Beispiel 1 zum Animpfen verwendet.

Nach 17 Stunden Fermentationszeit wird mit der Zugabe von 2-Methylbutanol über eine Pumpe begonnen. Die Dosierung des Substrats wird über einen Flow-Controller gesteuert. 2-Methylbutanol wird gemäß folgendem Flow-Profil zugegeben:

| Fermentationszeit | Flußrate |
|---|---|
| 0 h | 0 g/lh |
| 17 h | 1,0 g/lh |
| 20 h | 4,0 g/lh |
| 28 h | 3,5 g/lh |
| 31 h | 3,0 g/lh |
| 35 h | 2,5 g/lh |
| 39 h | 2,0 g/lh |
| 45 h | 1,5 g/lh |
| 51 h | 1,0 g/lh |
| 55 h | 0 g/lh |

Während des Feedings wird der pH-Wert im Bereich von 6,2 ― 6,4 mit NH₄⁺ konstant gehalten.

Die Endkonzentration an 2-Methylbuttersäure beträgt laut HPLC-Analytik 80 g/l. Die molare Umsetzung beträgt knapp 89 %.

### Beispiel 5 - Herstellung von natürlicher Isovaleriansäure aus Isoamylalkohol

Es werden 125 g Mannit und 75 g Hefeextrakt in 9,9 l Wasser in einem 10 l Fermenter gelöst, 10 ml Antischaummittel hinzu gegeben und der pH-Wert auf 6,3 eingestellt. Das so hergestellte Medium wird für 30 Minuten bei 121°C sterilisiert.

Die Drehzahl des Rührers beträgt 500 Upm, die Belüftung 5 Nl/min; die Temperatur 30°C. Nach Einstellung dieser Parameter wird die Vorkultur gemäß Beispiel 1 zum Animpfen verwendet.

Nach 17 Stunden Fermentationszeit wird mit der Zugabe von Isoamylalkohol über eine Pumpe begonnen. Die Dosierung des Substrats wird über einen Flow-Controller gesteuert. Isobutanol wird gemäß folgendem Flow-Profil zugegeben:

| Fermentationszeit | Flussrate |
|---|---|
| 0 h | 0 g/lh |
| 17 h | 1,0 g/lh |
| 20 h | 4,0 g/lh |
| 28 h | 3,5 g/lh |
| 31 h | 3,0 g/lh |
| 35 h | 2,5 g/lh |
| 39 h | 2,0 g/lh |
| 44 h | 1,5 g/lh |
| 48 h | 1,0 g/lh |
| 49,5 h | 0 g/lh |
| 55 h | 1,0 g/lh |
| 58 h | 0 g/lh |
| 63 h | 1,0 g/lh |
| 66 h | 0 g/lh |

Während des Feedings wird der pH-Wert im Bereich von 6,2 ― 6,4 mit NH₄⁺ konstant gehalten.

Die Fermentation ist nach 70,5 Stunden beendet. Die Endkonzentration an Isovaleriansäure beträgt nach Aufarbeitung der Fermentationslösung 82 g/l. Die molare Umsetzung beträgt knapp 85 %.

### Beispiel 6 - Herstellung von natürlicher Propionsäure aus n-Propanol

Es werden 125 g Mannit und 75 g Hefeextrakt in 9,9 l Wasser in einem 10 l Fermenter gelöst, 10 ml Antischaummittel hinzu gegeben und der pH-Wert auf 6,3 eingestellt. Das so hergestellte Medium wird für 30 Minuten bei 121°C sterilisiert.

Die Drehzahl des Rührers beträgt 500 Upm, die Belüftung 5 Nl/min; die Temperatur 30°C. Nach Einstellung dieser Parameter wird die Vorkultur gemäß Beispiel 1 zum Animpfen verwendet.

Nach 17 Stunden Fermentationszeit wird mit der Zugabe von Propanol über eine Pumpe begonnen. Die Dosierung des Substrats wird über einen Flow-Controller gesteuert. Isobutanol wird gemäß folgendem Flow-Profil zugegeben:

| Fermentationszeit | Flussrate |
|---|---|
| 0 h | 0 g/lh |
| 17 h | 1,0 g/lh |
| 20 h | 3,5 g/lh |
| 27 h | 3,0 g/lh |
| 29 h | 2,0 g/lh |
| 35 h | 1,5 g/lh |
| 60 h | 1,0 g/lh |
| 82 h | 0 g/lh |
| 87 h | 1,0 g/lh |
| 90 h | 0 g/lh |

Während des Feedings wird der pH-Wert im Bereich von 6,2 ― 6,4 mit NH₄⁺ konstant gehalten.

Die Fermentation ist nach 92 Stunden beendet. Die Endkonzentration an Propionsäure beträgt laut HPLC-Analytik 94 g/l. Die molare Umsetzung beträgt 88,3%.

### Beispiel 7 - Herstellung von natürlicher Phenylessigsäure aus Phenylethanol

Es werden 125 g Mannit und 75 g Hefeextrakt in 9,9 l Wasser in einem 10 l Fermenter gelöst, 10 ml Antischaummittel hinzu gegeben und der pH-Wert auf 6,3 eingestellt. Das so hergestellte Medium wird für 30 Minuten bei 121°C sterilisiert.

Die Drehzahl des Rührers beträgt 500 Upm, die Belüftung 5 Nl/min; die Temperatur 30°C. Nach Einstellung dieser Parameter wird die Vorkultur gemäß Beispiel 1 zum Animpfen verwendet.

Nach 17 Stunden Fermentationszeit wird mit der Zugabe von Phenylethylalkohol über eine Pumpe begonnen. Die Dosierung des Substrats wird über einen Flow-Controller gesteuert. Phenylethylalkohol wird gemäß folgendem Flow-Profil zugegeben:

| Fermentationszeit | Flussrate |
|---|---|
| 0 h | 0 g/lh |
| 17 h | 1,0 g/lh |
| 20 h | 4,0 g/lh |
| 23,5 h | 2,0 g/lh |
| 24 h | 2,5 g/lh |
| 30 h | 2,0 g/lh |
| 37 h | 1,5 g/lh |
| 41 h | 1,0 g/lh |
| 43,8 h | 0 g/lh |
| 50,5 h | 1,0 g/lh |
| 53 h | 0 g/lh |
| 58 h | 1,0 g/lh |
| 60 h | 0 g/lh |
| 65 h | 1,0 g/lh |
| 67 h | 0 g/lh |

Während des Feedings wird der pH-Wert im Bereich von 6,2 - 6,4 mit NH₄⁺ konstant gehalten.

Die maximale Produktkonzentration wird nach 48 Stunden erreicht. Die Konzentration an Phenylessigsäure beträgt laut HPLC-Analytik 54 g/l. Die molare Umsetzung beträgt 88,5 %.

Bei Übertragung des Prozesses auf den 200 l Maßstab wurden 52 g/l erhalten; die molare Umsetzung lag hier bei 95 %.

### Beispiel 8 - Herstellung von natürlicher Benzoesäure aus Benzylalkohol

Es werden 125 g Mannit und 75 g Hefeextrakt in 9,9 l Wasser in einem 10 l Fermenter gelöst, 10 ml Antischaummittel hinzu gegeben und der pH-Wert auf 6,3 eingestellt. Das so hergestellte Medium wird für 30 Minuten bei 121°C sterilisiert.

Die Drehzahl des Rührers beträgt 500 Upm, die Belüftung 5 Nl/min; die Temperatur 30°C. Nach Einstellung dieser Parameter wird die Vorkultur gemäß Beispiel 1 zum Animpfen verwendet.

Nach 21,25 Stunden Fermentationszeit wird mit der Zugabe von Benzylalkohol über eine Pumpe begonnen. Die Dosierung des Substrats wird über einen Flow-Controller gesteuert. Benzylalkohol wird gemäß folgendem Flow-Profil zugegeben:

| Fermentationszeit | Flussrate |
|---|---|
| 0 h | 0 g/lh |
| 21,25 h | 1,0 g/lh |
| 23 h | 3,0 g/lh |
| 28 h | 2,5 g/lh |
| 31 h | 2,0 g/lh |
| 34 h | 1,5 g/lh |
| 37 h | 1,0 g/lh |
| 40 h | 0 g/lh |
| 43 h | 1,0 g/lh |
| 47,5 h | 0 g/lh |
| 50,5 h | 1,0 g/lh |
| 54 h | 0 g/lh |
| 57 h | 1,0 g/lh |
| 60 h | 0 g/lh |
| 63 h | 1,0 g/lh |
| 65 h | 0 g/lh |

Während des Feedings wird der pH-Wert im Bereich von 6,2 ― 6,4 mit NH₄⁺ konstant gehalten.

Die Fermentation ist nach 68 Stunden beendet. Die Endkonzentration an Benzoesäure beträgt laut HPLC-Analytik 51 g/l. Das Edukt wurde nahezu quantitativ umgesetzt.

### Beispiel 9 - Herstellung von natürlicher Zimtsäure aus Zimtalkohol

Es werden 125 g Mannit und 75 g Hefeextrakt in 9,9 l Wasser in einem 10 l Fermenter gelöst, 10 ml Antischaummittel hinzu gegeben und der pH-Wert auf 6,3 eingestellt. Das so hergestellte Medium wird für 30 Minuten bei 121°C sterilisiert.

Die Drehzahl des Rührers beträgt 500 Upm, die Belüftung 5 Nl/min; die Temperatur 30°C. Nach Einstellung dieser Parameter wird die Vorkultur gemäß Beispiel 1 zum Animpfen verwendet.

Nach 17 Stunden Fermentationszeit wird mit der Zugabe von Zimtalkohol über eine Pumpe begonnen. Um den Zimtalkohol in der flüssigen Phase zu haben, wurde das Edukt erwärmt. Zimtalkohol wird gemäß folgendem Flow-Profil zugegeben:

| Fermentationszeit | Flussrate |
|---|---|
| 0 h | 0 g/lh |
| 17 h | 1,2 g/lh |
| 20 h | 2,4 g/lh |
| 21 h | 3,6 g/lh |
| 25,25 h | 0 g/lh |
| 26,25 h | 2,4 g/lh |
| 28 h | 1,2 g/lh |
| 30 h | 0 g/lh |
| 31 h | 1,2 g/lh |
| 32 h | 0 g/lh |

Die Fermentation ist nach 44 Stunden beendet. Die Endkonzentration an Zimtsäure beträgt laut HPLC-Analytik 27 g/l. Das Edukt wurde nahezu quantitativ umgesetzt.

### Beispiel 10 Herstellung von natürlicher 3-Methylthiopropionsäure aus 3-Methylthiopropanol

Es werden 125 g Mannit und 125 g Hefeextrakt in 10 l Wasser in einem 10 l Fermenter gelöst, 10 ml Antischaummittel hinzu gegeben und der pH-Wert auf 6,3 eingestellt. Das so hergestellte Medium wird für 30 Minuten bei 121°C sterilisiert.

Die Drehzahl des Rührers beträgt 500 Upm, die Belüftung 5 Nl/min; die Temperatur 27°C. Nach Einstellung dieser Parameter wird die Vorkultur gemäß Beispiel 1 zum Animpfen verwendet.

Nach 16 Stunden Fermentationszeit wird mit der Zugabe von 3-Methylthiopropanol über eine Pumpe begonnen. Die Dosierung des Substrats erfolgt gemäß folgendem Flow-Profil:

| Fermentationszeit | Flussrate |
|---|---|
| 0 h | 0 g/lh |
| 16 h | 1,0 g/lh |
| 17,66 h | 2,0 g/lh |
| 19 h | 3,0 g/lh |
| 20,8 h | 4,2 g/lh |
| 21,8 h | 4,8 g/lh |
| 23,2 h | 4,2 g/lh |
| 23,8 h | 2,6 g/lh |
| 42,5 h | 2,2 g/lh |
| 48,8 h | 0 g/lh |

Während des Feedings wird der pH-Wert im Bereich von 6,2 ― 6,4 mit NH₄⁺ konstant gehalten.

Die Fermentation ist nach 65 Stunden beendet. Die Endkonzentration an 3-Methylthiopropionsäure beträgt laut HPLC-Analytik 82,6 g/l. Die molare Umsetzung beträgt fast 100 %.

### Beispiel 12 Vergleich der Raum-Zeit-Ausbeute des erfindungsgemäßen Verfahrens mit bekannten Verfahren:

Im folgenden Beispiel werden die Raum-Zeit-Ausbeuten der neuen Produktionsverfahrens mit dem Gluconobacter sp. DSM 12884 mit den bisher bekannten Verfahren verglichen, um die Überlegenheit des neuen Prozesses zu verdeutlichen.

| Produkt | Prozessdauer [Stunden] | Produktkonzentration [g/l] | Raum-Zeit-Ausbeute [g/l/Std.] | Prozess |
|---|---|---|---|---|
| Buttersäure | 120 | 13 | 0,11 | DE-A 37 13 668 |
| Buttersäure | 60 | 39,3 | 0,66 | J. Chem. Tech. Biotechnol. 1997, 68, 214-218 |
| Buttersäure | 80 | 49 | 0,61 | DE-A 195 03 598 |
| Buttersäure | 90 | 60 | 0,67 | J. Chem. Tech. Biotechnol. 1997, 70, 294-298 |
| Buttersäure | 73 | 95 | 1,3 | Erfindung |
| Propionsäure | 70 | 43,7 | 0,62 | DE-A 195 03 598 |
| Propionsäure | 90 | 60 | 0,67 | J. Chem. Tech. Biotechnol. 1997, 70, 294-298 |
| Propionsäure | 92 | 94 | 1,02 | Erfindung |
| Isobuttersäure | 42 | 21 | 0,5 | DE-A 37 13 668 |
| Isobuttersäure | 74 | 92,7 | 1,25 | Erfindung |
| Isovaleriansäure | 25 | 17 | 0,68 | DE-A 37 13 668 |
| Isovaleriansäure | 90 | 45 | 0,5 | J. Chem. Tech. Biotechnol. 1997, 70, 294-298 |
| Isovaleriansäure | 70,5 | 82 | 1,16 | Erfindung |
| 2-Methylbuttersäure | 24 | 7 | 0,29 | DE-A 37 13 668 |
| 2-Methylbuttersäure | 90 | 44 | 0,49 | J. Chem. Tech. Biotechnol. 1997, 70, 294-298 |
| 2-Methylbuttersäure | 52 | 80 | 1,54 | Erfindung |

Wie aus der Tabelle zu ersehen ist, ist mit dem neuen Prozess mit Gluconobacter sp. DSM 12884 bei den schon beschriebenen Alkoholoxidationen eine enorme Steigerung sowohl der Raum-Zeit-Ausbeute als auch in der absoluten Produktkonzentration zu verzeichnen. So erhöht sich bei Buttersäure die Raum-Zeit-Ausbeute um 94 % und die Produktkonzentration um 58 % gegenüber dem bisher besten Prozess. Bei Propionsäure erhöht sich die Raum-Zeit-Ausbeute um 52 % und die Produktkonzentration um 57 %. Noch höher sind die Steigerungen bei Isobuttersäure mit 150 % bei der Raum-Zeit-Ausbeute und 341 % bei der Produktkonzentration. Die Steigerungen für Isovaleriansäure und 2-Methylbuttersäure sind 132 % und 82 %, bzw. 214 % und 82 %.

## Patentansprüche

1. Verfahren zur Herstellung von aliphatischen, aromatischen und Thio-Carbonsäuren in Bioreaktoren, dadurch gekennzeichnet, dass Bakterien der Gattung Gluconobacter enthaltende Kulturen eingesetzt werden.

2. Verfahren nach Anspruch 1 dadurch gekennzeichnet, dass eine aus Bakterien der Gattung Gluconobacter bestehende Reinkultur eingesetzt wird.

3. Verfahren nach einem der Ansprüche 1 oder 2 dadurch gekennzeichnet, dass Gluconobacter sp. HR 101 (DSM 12884) eingesetzt wird.

4. Verfahren nach einem der Ansprüche 1 bis 3 dadurch gekennzeichnet, dass synthetische, halbsynthetische oder komplexe Substrate eingesetzt werden.

5. Verfahren nach einem der Ansprüche 1 bis 4 dadurch gekennzeichnet, dass kohlenstoffhaltige sowie stickstoffhaltige Verbindungen, anorganische Salze, Spurenelemente und/oder Vitamine enthaltende Substrate eingesetzt werden.

6. Verfahren nach Anspruch 5 dadurch gekennzeichnet, dass das Substrat als kohlenstoffhaltige Verbindungen Zucker, Zuckeralkohole, Alkohol, organische Säuren, komplexe Gemische, Öle oder Gemische zweier oder mehrerer dieser Substanzen enthält.

7. Verfahren nach Anspruch 6 dadurch gekennzeichnet, dass Glucose, Glyzerin, Mannit, Zitronensäure, Malzextrakt, Hefeextrakt, Casein, Caseinhydrolysat und Rizinusöl oder Gemische zweier oder mehrerer dieser Substanzen enthaltende Substrate eingesetzt werden.

8. Verfahren nach einem der Ansprüche 1 bis 7 dadurch gekennzeichnet, dass anorganische Verbindungen und/oder organische Verbindungen enthaltenden Substrate eingesetzt werden.

9. Verfahren nach Anspruch 8 dadurch gekennzeichnet, dass Nitrate, Ammoniumsalze, Hefeextrakt, Sojamehl, Baumwollsaatmehl, Casein, Caseinhydrolysat, Weizengluten und Maisquellwasser eingesetzt werden.

10. Verfahren nach einem der Ansprüche 1 bis 9 dadurch gekennzeichnet, Sulfate, Nitrate, Chloride, Carbonate und Phosphate der Metalle Natrium, Kalium, Magnesium, Mangan, Calcium, Zink und Eisen oder Gemische zweier oder mehrerer dieser Verbindungen enthaltende Substrate eingesetzt werden.

11. Verfahren nach einem der Ansprüche 1 bis 10 dadurch gekennzeichnet, dass die Temperaturen im Bereich von 10 bis 40°C liegen.

12. Verfahren nach einem der Ansprüche 1 bis 11 dadurch gekennzeichnet, dass der pH-Wert im Bereich von 4 bis 8 liegt.

13. Gluconobacter sp. HR 101 (DSM 12884).

14. Verwendung von Gluconobacter sp. nach Anspruch 13 zur Herstellung von aliphatischen, aromatischen und Thiocarbonsäuren.

15. Verfahren zur Herstellung von aliphatischen, aromatischen und Thiocarbonsäuren dadurch gekennzeichnet, dass die molare Umsetzung der Substrate größer als 60 % ist.

16. Verfahren zur Herstellung von natürliche Buttersäure in Bioreaktoren, dadurch gekennzeichnet, dass Bakterien der Gattung Gluconobacter enthaltende Kulturen eingesetzt werden.

17. Verfahren zur Herstellung von Isobuttersäure in Bioreaktoren dadurch gekennzeichnet, dass Bakterien der Gattung Gluconobacter enthaltende Kulturen eingesetzt werden.

18. Verfahren zur Herstellung von Isovaleriansäure in Bioreaktoren, dadurch gekennzeichnet, dass Bakterien der Gattung Gluconobacter enthaltende Kulturen eingesetzt werden.

19. Verfahren zur Herstellung von 2-Methylbuttersäure in Bioreaktoren, dadurch gekennzeichnet, dass Bakterien der Gattung Gluconobacter enthaltende Kulturen eingesetzt werden.

20. Verfahren zur Herstellung von Propionsäure in Bioreaktoren, dadurch gekennzeichnet, dass Bakterien der Gattung Gluconobacter enthaltende Kulturen eingesetzt werden.

21. Verfahren zur Herstellung von Benzoesäure in Bioreaktoren, dadurch gekennzeichnet, dass Bakterien der Gattung Gluconobacter enthaltende Kulturen eingesetzt werden.

22. Verfahren zur Herstellung von Phenylessigsäure in Bioreaktoren, dadurch gekennzeichnet, dass Bakterien der Gattung Gluconobacter enthaltende Kulturen eingesetzt werden.

23. Verfahren zur Herstellung von Zimtsäure in Bioreaktoren, dadurch gekennzeichnet, dass Bakterien der Gattung Gluconobacter enthaltende Kulturen eingesetzt werden.

24. Verfahren zur Herstellung von 3-Methylthiopropionsäure in Bioreaktoren, dadurch gekennzeichnet, dass Bakterien der Gattung Gluconobacter enthaltende Kulturen eingesetzt werden.

25. Verfahren nach den Ansprüchen 16 bis 24, dadurch gekennzeichnet, dass eine aus Bakterien der Gattung Gluconobacter bestehende Reinkultur eingesetzt wird.

26. Verfahren nach den Ansprüchen 16 bis 24, dadurch gekennzeichnet, dass Gluconobacter sp. HR 101 (DSM 12884) eingesetzt wird.
